# EUROPÄISCHE PATENTANMELDUNG

(11) **EP 2 511 687 A2**
(43) Veröffentlichungstag der Anmeldung: **17.10.2012**
(21) Anmeldenummer: 12164133.6
(22) Anmeldetag: 13.04.2012
(51) Int. Cl.: G01N 1/14, G01N 1/40

(54) **Verfahren und Vorrichtung zur Festphasenextraktion aus einer Flüssigkeit**

(30) Priorität: 14.04.2011 DE 102011007392
(71) Anmelder: Helmholtz-Zentrum für Umweltforschung GmbH-UFZ, 04318 Leipzig (DE); MAXX Mess- und Probenahmetechnik GmbH, 72414 Rangendingen (DE)
(72) Erfinder: Schulze, Tobias, 12047 Berlin (DE); Brack, Werner, 04175 Leipzig (DE); Walz, Karl-Heinz, 72379 Hechingen - Weilheim (DE)
(74) Vertreter: Gulde Hengelhaupt Ziebig & Schneider

(57) **Zusammenfassung**

Verfahren und Vorrichtung zur großvolumigen Festphasenextraktion aus einer Flüssigkeit, wobei mit der Vorrichtung die Flüssigkeit einer ersten Extraktion mittels eines Sorbens unterzogen wird, danach die Flüssigkeit angesäuert oder alkalisiert wird, und die angesäuerte oder alkalisierte Flüssigkeit einer zweiten Extraktion mittels eines Sorbens unterzogen wird.

## Beschreibung

Die vorliegende Erfindung betrifft ein Verfahren sowie eine Vorrichtung zur großvolumigen Festphasenextraktion aus einer Flüssigkeit. Insbesondere betrifft die Erfindung Verfahren und Vorrichtung zur Festphasenextraktion vor Ort von Stoffen und Verbindungen z.B. aus Gewässern, Grundwassermessstellen oder Abwasserströmen unter Entnahme großvolumiger Proben.

Zur Erkennung etwaiger Schadwirkungen von in Gewässern, Grundwässern und Abwässern enthaltenen chemischen Stoffen auf Ökosysteme und Trinkwasserressourcen und - damit auch - auf die menschliche Gesundheit ist die Überwachung und Analyse von Gewässer-, Grundwasser- und Abwasserproben zwingend notwendig. Dazu eignen sich besonders mobile und automatisierte Probenehmer, mit denen aktiv vor Ort organische Schadstoffe in Gewässern, Grundwassermessstellen und Kläranlagenabläufen gesammelt werden können und für analytische Zwecke zur Verfügung gestellt werden können. Dabei sind stichprobenartige Entnahmen von diskreten Wasserproben im Hinblick auf ihre zeitliche Repräsentativität kritisch zu bewerten, weil die Zusammensetzung der Proben nur dem Gehalt an chemischen Stoffen entspricht, den die Probe bzw. das Gewässer, Grundwasser oder Abwasser während der relativ kurzen Zeit der Probenahme aufwies. Es hat sich außerdem herausgestellt, dass für gewöhnlich mindestens 100 Liter Wasser notwendig sind, um mit biologisch- und chemischanalytischen Methoden bislang unbekannte toxische Spurenstoffe (z. B. Transformationsprodukte von Pflanzenschutzmitteln) zu identifizieren und zu isolieren sowie deren Struktur und potentielle Schadwirkung aufzuklären. Nachteilig daran ist allerdings, dass nach Stand der Technik ein zeit- und kostenaufwändiger Transport zum Labor notwendig ist, währenddessen die Probe der Gefahr einer Veränderung - und damit Verlust von Zielsubstanzen - ausgesetzt ist und dieser zudem personelle Leistung bindet. Die am Markt befindlichen Probenehmer können in der Regel nur bis ca. 60 Liter an Probe lagern und sind so ausgeführt, dass durch die mit den Proben in Berührung kommenden Materialien (z. B. PVC, ABS, und Silikon) die Besorgnis einer Probenverfälschung und Verlust von Analyten besteht.

Mit Passivsammlern nach DIN EN ISO 5667-23 ist für gewöhnlich nicht ein ausreichend großes Flüssigkeitsvolumen auswertbar. Zudem ergibt sich das Problem, dass für gewöhnlich keine Rückschlüsse auf die tatsächlichen Konzentrationen von chemischen Stoffen in der Probe möglich sind, für die keine zeitaufwändige Kalibrierung zur Bestimmung sogenannter Sammelraten vorliegt. Außerdem lässt die hohe Anzahl von zu analysierenden Komponenten und die daraus möglicherweise vorliegenden Kombinationen die Verwendung von Passivsammlern als ungeeignet erscheinen, da Passivsammler aus chemisch-physikalischen Gründen nur für spezifische Gruppen nichtpolarer oder polarer Verbindungen zur Verfügung stehen. Dissoziierte bzw. ionische Verbindungen werden nach Stand der Technik nur unzureichend erfasst.

Ein Verfahren und eine Vorrichtung zur Probenahme und Festphasenextraktion ist aus dem US-Patent 5,844,147 bekannt. In diesem Dokument ist beschrieben, dass eine Wasserprobe relativ schnell entnommen wird, unter Druck in einer Druckkammer gespeichert wird und mit relativ geringer Geschwindigkeit durch ein Festphasenextraktionsgerät geleitet wird.

Nachteilig hierbei erscheint jedoch, dass das Festphasenextraktionsgerät nur zur Extraktion bestimmter chemischer Verbindungen bzw. Stoffe ausgestaltet ist, so dass die Analysemöglichkeiten zwangsläufig begrenzt sind. Des Weiteren wird hier eine Schlauchpumpe zur Förderung der Probe genutzt. Der Schlauch besteht in der Regel aus Silikon und ist daher aufgrund einer möglichen Absorption der Analyten sehr kritisch im Hinblick auf eine Probenverfälschung.

Der vorliegenden Erfindung liegt somit die Aufgabe zugrunde, ein Verfahren sowie eine Vorrichtung zur Verfügung zu stellen, mittels derer in einfacher, kostengünstiger, zuverlässiger und flexibler Weise die Extraktion unterschiedlicher Verbindungen bzw. Stoffe aus einer Flüssigkeitsprobe, insbesondere aus einer Flüssigkeitsprobe mit großem Volumen, möglich ist.

Diese Aufgabe wird durch das erfindungsgemäße Verfahren zur großvolumigen Festphasenextraktion aus einer Flüssigkeit gemäß Anspruch 1 sowie durch die erfindungsgemäße Vorrichtung zur Festphasenextraktion aus einer Flüssigkeit gemäß Anspruch 8 gelöst. Vorteilhafte Ausgestaltungen des erfindungsgemäßen Verfahrens sind in den Unteransprüchen 2 bis 7 angegeben. Vorteilhafte Ausgestaltungen der Vorrichtung sind in den Unteransprüchen 9 bis 114 angegeben. Ergänzend wird erfindungsgemäß eine Verwendung der erfindungsgemäßen Vorrichtung zur Festphasenextraktion in Anspruch 15 zur Verfügung gestellt.

Das erfindungsgemäße Verfahren zur Festphasenextraktion aus einer Flüssigkeit wird derart durchgeführt, dass die Flüssigkeit einer ersten Extraktion mittels Sorbens unterzogen wird, danach der pH-Wert der Flüssigkeit erniedrigt oder erhöht wird, und die angesäuerte oder alkalisierte Flüssigkeit einer zweiter Extraktion mittels eines zweiten Sorbens unterzogen wird. Die Art des Sorbens wird hierbei so gewählt, dass dieses eine hohe Affinität zu den chemisch-physikalischen Eigenschaften der zu extrahierenden organischen Stoffe oder Verbindungen aufweist.

Flüssigkeitsproben werden z.B. Gewässern oder Abwasserströmen mit großen Volumina, vorzugsweise mindestens 100 Litern, entnommen. Die in den Flüssigkeitsproben gegebenenfalls vorhandenen Schwebstoffe können z.B. durch Filtration abgeschieden werden und die gelöst vorliegenden Stoffe und Verbindungen werden extrahiert und können gebunden an die Sorbentien ins Labor transportiert und den jeweiligen Analysen zugeführt werden. Dabei weisen die Flüssigkeitsproben in der ersten Extraktion einen ersten pH-Wert auf. Bei Untersuchung von Gewässer-und Abwasserproben ist anzunehmen, dass dieser erste pH-Wert neutral ist. Vorzugsweise liegt er bei einem pH-Wert zwischen 6 und 8. Nach Änderung des pH-Wertes durch z.B. Ansäuern oder Alkalisieren weist die Flüssigkeit in der zweiten Extraktion einen zweiten pH-Wert auf, der saurer oder basischer ist als der erste pH-Wert. Der Vorteil des erfindungsgemäßen Verfahrens liegt insbesondere darin, dass einige Verbindungen bei normalem pH-Wert in dissoziierter bzw. ionischer Form vorliegen können, in Abhängigkeit von der jeweiligen Säure- oder Basenkonstante. Sie können deswegen gegebenenfalls nicht mittels eines Sorbens aus der Flüssigkeit extrahiert werden. Durch Ansäuern oder Alkalisieren und die damit verbundene pH-Wert-Änderung liegen diese Verbindungen nicht mehr in dissoziierter Form bzw. in ionischer Form vor und können somit an das Sorbens binden.

Zur Festphasenextraktion von Stoffen und vorzugsweise organischen Verbindungen aus der Flüssigkeitsprobe ist somit in jeder der Extraktionseinrichtungen ein Sorbens vorgesehen. In diesem Sorbens reichern sich die in der Flüssigkeit gelösten Stoffe und Verbindungen an.

Innerhalb der ersten und/oder zweiten Extraktion und dort jeweils vor der mittels eines Sorbens durchgeführten Extraktion wird die Flüssigkeit - vorzugsweise mittels einer Filterkerze - gegebenenfalls gefiltert. Dies dient der Entfernung von in der Flüssigkeit enthaltenen Schwebstoffen.

Zusätzlich oder alternativ kann vorgesehen sein, dass vor der ersten Filtration und Extraktion eine Abscheidung von in der Flüssigkeit enthaltenen absetzbaren Stoffen und/ oder Schwebstoffen erfolgt. Die Abscheidung von absetzbaren Stoffen erfolgt bevorzugt in einem Entspannungsgefäß, welches die Sauggeschwindigkeit in der Ansaugleitung reduziert und somit zu einem Absetzen der absetzbaren Stoffe führt. Das Entfernen von Schwebstoffen erfolgt in nachgeschalteten Filterkerzen und Filterkartuschen zur Feinfilterung.

Als bevorzugte Ausführungsform des Verfahrens ist vorgesehen, dass die Flüssigkeit Wasser ist und diese in der ersten Filterung einen pH-Wert von 6 bis 8, vorzugsweise 7, aufweist.

Es kann insbesondere vorgesehen sein, dass nach der ersten Filtration und Extraktion eine Bestimmung des pH-Wertes der Flüssigkeit durchgeführt wird und durch Ansäuern oder Alkalisieren der Flüssigkeit ein bestimmter pH-Wert im sauren oder basischen Bereich eingestellt wird. Es ist dabei bevorzugt ein bestimmter pH-Wert einzustellen, bei dem anzunehmen ist, dass bestimmte Verbindungen nicht in dissoziierter Form bzw. ionischer Form vorliegen und somit an das Sorbens binden können.

Erfindungsgemäß wird außerdem eine Vorrichtung zur Festphasenextraktion aus einer Flüssigkeit zur Verfügung gestellt, die eine erste Einrichtung zur Extraktion der Flüssigkeit mittels eines Sorbens, eine Einrichtung zur automatisierten Messung sowie Erhöhung oder Erniedrigung des pH-Werts der Flüssigkeit sowie eine zweite Einrichtung zur Extraktion der Flüssigkeit mittels eines Sorbens umfasst. Die erste Einrichtung zur Extraktion der Flüssigkeit ist dabei strömungstechnisch mit der Einrichtung zur Erhöhung oder Erniedrigung des pH-Werts und diese wiederum strömungstechnisch mit der zweiten Einrichtung zur Extraktion der Flüssigkeit verbunden. Demzufolge kann die Flüssigkeit von der ersten Extraktionseinrichtung über die Einrichtung zur Erhöhung oder Erniedrigung des pH-Werts in die zweite Extraktionseinrichtung strömen. Die erfindungsgemäße Vorrichtung dient insbesondere zur Durchführung des erfindungsgemäßen Verfahrens, wobei die erste Extraktionseinrichtung zur Durchführung der ersten Extraktion dient und die zweite Extraktionseinrichtung zur Durchführung der zweiten Extraktion dient. Das heißt, dass die erste sowie auch die zweite Einrichtung zur Extraktion der Flüssigkeit ein Sorbens zur Festphasenextraktion aufweist. Zur Entfernung von Schwebstoffen ist bevorzugt vorgesehen, dass die erste Einrichtung zur Extraktion der Flüssigkeit in Strömungsrichtung vor dem Sorbens eine Filterkerze zur Entfernung von Schwebstoffen aufweist. Diese Filterkerze ist strömungstechnisch mit der nachgeschalteten, ein Sorbens aufweisenden Extraktionseinrichtung verbunden.

Ebenfalls zur Abscheidung von absetzbaren Stoffen aus der Flüssigkeit kann in Strömungsrichtung vor wenigstens einer der Extraktionseinrichtungen eine Entspannungseinrichtung angeordnet sein. Auch diese Entspannungseinrichtung ist strömungstechnisch mit der jeweiligen nachgeschalteten Extraktionseinrichtung verbunden. Die Entspannungseinrichtung kann z. B. ein Entspannungsgefäß sein, in dem sich absetzbare Stoffe nach unten absetzen.

In vorteilhafter Ausgestaltung umfasst die erfindungsgemäße Vorrichtung in Strömungsrichtung nach der ersten Filtereinrichtung eine Mischeinrichtung. Diese Mischeinrichtung ist mit einer Dosiereinrichtung strömungstechnisch verbunden, die zur Zugabe von Säure oder Base in die Mischeinrichtung eingerichtet ist. Damit lässt sich in einfacher und automatisierter Weise eine Säure oder Base in die zu extrahierende Flüssigkeit einbringen und deren pH-Wert verändern.

Zur Filterung unterschiedlicher Lösungen oder Stoffe ist vorteilhafter Weise vorgesehen, dass die erste Extraktionseinrichtung eine Filterkartusche zur Feinfilterung und eine mit dem Sorbens zumindest teilweise befüllte Kartusche zur Festphasenextraktion aufweist. Durch die Filterkartusche zur Feinfilterung lassen sich Partikel mit Durchmesser von größer als 0,45 µm filtern. Das Sorbens ist in einer weiteren Kartusche angeordnet. Eine derartige Sorbenskartusche kann auch die Extraktion in der zweiten Extraktionseinrichtung übernehmen.

Zur Vermeidung des Trockenfallens der jeweiligen Extraktionseinrichtung ist vorgesehen, dass die erste und/oder zweite Extraktionseinrichtung eine Zurückhalteeinrichtung zur Zurückhaltung von Flüssigkeit in der Extraktionseinrichtung aufweist, die verhindert, dass unter Umgebungsdruck stehende Flüssigkeit in der Extraktionseinrichtung unter die geodätische Höhe des höchsten Punktes eines Filters oder einer Extraktionskartusche, insbesondere des höchsten Filters oder der höchsten Extraktionskartusche, der Extraktionseinrichtung absinkt. Dieser Filter kann auch das Sorbens sein. Bei Ausgestaltung der Zurückhalteeinrichtung als Siphonauslauf verhindert dieser durch seine Erstreckung auf eine geodätische Höhe, die höher ist als die des höchsten Punktes der Extraktionseinrichtung, dass unter Umgebungsdruck stehende Flüssigkeit aus den Filtern bzw. aus dem Sorbens strömen kann. Dadurch sind die Filter und Extraktionskartuschen ständig mit Flüssigkeit beaufschlagt und können nicht austrocknen.

Als weitere Vorteilhafte Ausgestaltung ist vorgesehen, dass die erste und/oder zweite Extraktionseinrichtung eine Füllstandserfassungseinheit aufweist, mittels derer in der Extraktionseinrichtung ein bestimmter Füllstand der Flüssigkeit detektierbar ist. Dies dient dazu, dass, wenn der bestimmte Füllstand der Flüssigkeit erreicht ist, die in der jeweiligen Extraktionseinrichtung befindliche Flüssigkeit mit einem nächsten, zu filternden Flüssigkeitsvolumen ausgetauscht wird. Dadurch wird erreicht, dass keine Luft in das System eindringt.

Ergänzend wird außerdem erfindungsgemäß eine Verwendung der Vorrichtung zur Festphasenextraktion zur Gewinnung von Wasserproben aus Gewässern und Abwässern sowie zur Extraktion von Substanzen aus den Proben zwecks Analyse zur Verfügung gestellt.

Die vorliegende Erfindung wird im Folgenden anhand des in der beiliegenden Zeichnung dargestellten Ausführungsbeispiels erläutert.

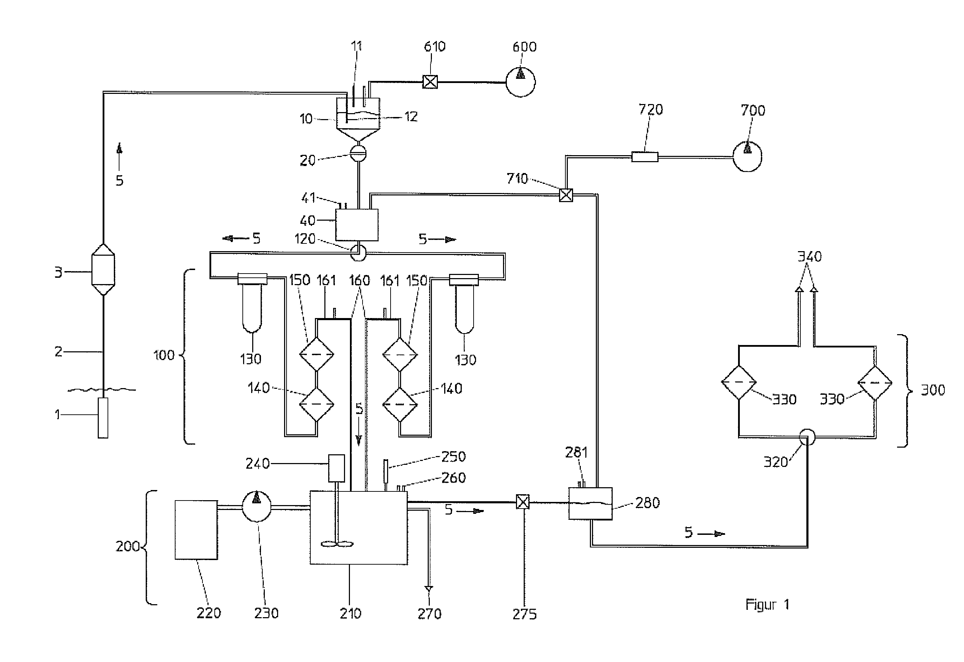

Es zeigt die einzige Figur 1 eine erfindungsgemäße Vorrichtung zur großvolumigen Festphasenextraktion und somit zur Durchführung des erfindungsgemäßen Verfahrens zur großvolumigen Festphasenextraktion aus einer Flüssigkeit.

Die Durchführung des Verfahrens wird anhand der in der Vorrichtung ablaufenden Vorgänge beschrieben.

Über ein Siebkorb 1 wird eine Flüssigkeitsprobe einem Gewässer, einer Grundwassermessstelle oder einem Kläranlagenablauf entnommen. Über die Zuleitung 2 gelangt die Flüssigkeitsprobe in eine Entspannungseinrichtung 3, welche zum Beispiel ein Entspannungsgefäß sein kann. Dort können sich absetzbare Stoffe absetzen. Danach strömt die Flüssigkeitsprobe strömt in Strömungsrichtung 5 in ein Dosiergefäß 10, in dem Vakuum herrscht, aufgrund dessen die Flüssigkeit angesogen wird. Die Flüssigkeit strömt über das Füllrohr 12 in das Dosiergefäß 10. Erreicht der Füllstand die Abschaltelektroden 11 an der Oberseite des Dosiergefäßes 10, wird statt Vakuum ein Überdruck im Dosiergefäß 10 erzeugt und Flüssigkeit durch das Füllrohr 12 wieder zurück in Richtung der Zuleitung 2 gedrückt, sodass die Zuleitung 2 entleert wird. Das Füllrohr 12 kann in der Höhe verstellt werden, sodass eine gewünschte Einzelprobenmenge im Dosiergefäß 10 genau eingestellt werden kann.

Anschließend wird der Absperrhahn 20 geöffnet. Die Flüssigkeit wird somit in den Behälter 40 abgelassen. Im Deckel dieses Behälters befinden sich 2 Leitfähigkeitselektroden 41 zur Erkennung von Flüssigkeit. Danach wird der Absperrhahn 20 wieder geschlossen. Wenn der Absperrhahn 20 wieder geschlossen ist wird über die Pumpe 700 und das Umschaltventil 710 Druck auf den Behälter 40 gegeben. Dies bewirkt, dass die aufgenommene Flüssigkeit durch die Öffnung des Kugelhahns 120 in die erste Einrichtung zur Extraktion 100 gedrückt wird. Dort strömt sie über die dargestellten doppelt ausgeführten Filterkerzen 130 jeweils in eine Filterkartusche zur Feinfilterung 140 und zu einem strömungstechnisch damit gekoppelten Sorbens 150. Sobald das Flüssigkeitsniveau im Behälter 40 unter das Niveau der Leitfähigkeitselektroden 41 sinkt, wird der Druckvorgang abgebrochen, sodass keine Flüssigkeit mehr aus dem Behälter herausgedrückt wird. In der Filterkartusche zur Feinfilterung 140 können Partikel mit einem Durchmesser von >0,45 µm herausgefiltert werden. Mittels des Sorbens wird eine Extraktion von in der Flüssigkeit vorliegenden Stoffen oder Verbindungen, insbesondere von im Wasser gelösten organischen Verbindungen, realisiert.

Mit dem Sorbens 150 ist eine Zurückhalteeinrichtung 160 strömungstechnisch gekoppelt. Der Siphonauslauf 161 der Zurückhalteeinrichtung erstreckt sich höher als das untere Niveau der Leitfähigkeitselektroden 41.

Der Siphonauslauf 161 gewährleistet, dass das Filtersystem immer mit Flüssigkeit beaufschlagt ist, sodass die Filter und das Sorbens nicht austrocknen können.

Dabei wird über Füllstandserfassungseinheit innerhalb des Behälters 40, die zum Beispiel durch Leitfähigkeitselektroden 41 ausgebildet sein kann, erfasst, ob der Füllstand der Flüssigkeit die Höhe der Füllstandserfassungseinheit 41 nach der Probenahme erreicht hat und nach dem Herausdrücken der Flüssigkeit wieder unter das Schaltniveau abgesunken ist. Dadurch wird das Eindringen von Luft in das Extraktionssystem verhindert sowie erreicht, dass immer genügend Flüssigkeit im System steht. Wird nun Flüssigkeit in den Behälter 40 geleitet, dient die Belüftung über Ventil 710 zum Entweichen von Luft.

Aus der beiliegenden Figur ist ersichtlich, dass die erste Einrichtung zur Extraktion 100 redundant ausgeführt ist, sodass selbst bei einer Verstopfung eines Filters oder des Sorbens noch der auf der jeweils anderen Seite angeordnete Filter beziehungsweise Sorbens die Funktion der Vorrichtung aufrecht erhalten kann. Gegebenenfalls sind Einrichtungen zur Umschaltung des Flüssigkeitsstroms vorgesehen, sodass automatisch oder manuell zwischen den beiden Extraktions-Strängen umgeschaltet werden kann.

Die die erste Einrichtung zur Extraktion 100 verlassene Flüssigkeit gelangt in die Einrichtung zum Ansäuern oder Alkalisieren 200. Diese umfasst eine Mischeinrichtung 210, in die die Flüssigkeit strömt. An die Mischeinrichtung 210 ist ein Säure- oder Basenbehälter 220 angeschlossen, aus dem mittels einer Dosiereinrichtung 230 Säure oder Base in die Mischeinrichtung 210 transportiert werden kann. In der Mischeinrichtung 210 wird mittels des Rührwerkes 240 eine ausreichende Durchmischung der Flüssigkeiten erzeugt. Das Rührwerk 240 kann so ausgeführt sein, dass sich in der Flüssigkeit ein Magnetrührstäbchen befindet und dieses von einem Magnetrührer angetrieben wird. An der Mischeinrichtung 210 ist weiterhin eine pH-Messeinrichtung 250 angeordnet, zur Feststellung des pH-Wertes des Flüssigkeitsgemischs in der Mischeinrichtung 210. Des Weiteren kann die Mischeinrichtung 210 mit einer Füllstandsanzeige 260 versehen sein.

Bei Erreichung einer bestimmten Füllstandshöhe in der Mischeinrichtung 210 oder nach einer bestimmten Anzahl von Probenahmezyklen wird; je nach gemessenem pH-Wert über die Dosiereinrichtung 230 Säure oder Base aus dem Säure- oder Basenbehälter 220 in die Mischeinrichtung 210 zugeführt und dort mittels des Rührwerkes 240 ein Gemisch hergestellt. Die pH-Messeinrichtung 250 ermittelt dabei den pH-Wert des Gemischs in der Mischeinrichtung 210. Ist ein vorher festgelegter pH-Wert im sauren Bereich erreicht, wird das Gemisch nach Öffnen von Ventil 275 in Strömungsrichtung 5 in einen Behälter 280 geleitet.

Um Beschädigungen des Systems zu vermeiden, ist an der Mischeinrichtung 210 außerdem ein Überlauf 270 angeordnet.

Vom Behälter 280 mit Elektroden 281 gelangt das Gemisch in gleicher Weise wie bei Behälter 40 beschrieben in die zweite Einrichtung zur Extraktion 300. Diese zweite Einrichtung zur Extraktion 300 umfasst ebenfalls einen Kugelhahn 320, mit dem der Volumenstrom über wenigstens ein Sorbens 330 zur Extraktion von weiteren Verbindungen aus der Flüssigkeit geleitet wird.

Diese zweite Einrichtung zur Extraktion 300 ist mit einem Überlauf 340 ausgestattet. Dadurch wird, wie oben bereits beschrieben, ein Trockenfallen des Sorbens 330 vermieden.

Zur Erzeugung der benötigten Druckverhältnisse in der Vorrichtung umfasst diese eine Membranpumpe 600 mit nachgeschaltetem Ventilsystem 610 zur Versorgung der Dosiereinheit 10 mit Druck bzw. Vakuum.

Eine zweite Membranpumpe 700 und ein Umschaltventil 710 sind zur Druckbeaufschlagung bzw. Belüftung der Behälter 40 bzw. 280 vorgesehen.

Durch das erwähnte Ansäuern oder Alkalisieren wird erreicht, dass einige Verbindungen, die bei neutralem pH-Wert der Flüssigkeit in dissoziierter beziehungsweise ionischer Form vorliegen können und daher durch die erste Einrichtung zur Extraktion 100 nicht aus der Flüssigkeit extrahiert werden können, durch die Änderung des pH-Wertes in nicht-dissoziierter beziehungsweise nichtionischer Form vorliegen und somit an das Sorbens 330 in der zweiten Einrichtung zur Extraktion 300 binden können.

Die erfindungsgemäße Vorrichtung kann über eine nicht dargestellte Steuereinrichtung und eine spezifische Software gesteuert werden, wobei die Datenübertragung in bevorzugter Ausführungsform über ein GSM-Modul oder über eine Internetschnittstelle erfolgt. Die Energieversorgung der Vorrichtung kann über einen Netzanschluss, eine Batterie oder eine Brennstoffzelle sowie über andere Energiequellen erfolgen.

Die erfindungsgemäße Vorrichtung und das erfindungsgemäße Verfahren eignen sich insbesondere zur Gewinnung von Stoffen und Verbindungen aus repräsentativen Wasserproben für die Anwendung bio-analytischer und chemischanalytischer Fragestellungen, insbesondere im Bereich des Gewässer-, Grundwasser- und Abwasserüberwachung. Die Vorrichtung ist insbesondere zur Anreicherung von gering konzentrierten Schadstoffen aus einer großen Wassermenge eingerichtet. Es lassen sich mit ihr mindestens 100 Liter vor Ort extrahieren, sodass ein Transport der entnommenen Wasserprobe ins Labor entfällt. Die Probenahme kann automatisch zeit-, volumen-, oder durchflussgesteuert über einen längeren Zeitraum, wie zum Beispiel über eine Woche, erfolgen. Damit lassen sich besonders repräsentative Aussagen über die Schadstoffkonzentration in Gewässern, Grundwässern und Abwässern erbringen.

Durch die Abtrennungsmöglichkeit von Sand und groben Schwebstoffen in der Entspannungseinrichtung 3 bzw. den Filtern 130 und Filterkartuschen 140 wird ein vorzeitiger Verschleiß der Vorrichtung verhindert. Die Dosierbarkeit der zuzuführenden Säure oder Base über die Dosiereinrichtung 230 erlaubt die flexible Einstellung unterschiedlicher pH-Werte in der Flüssigkeit, so dass in Abhängigkeit von der jeweiligen Säure- oder Basenkonstante unterschiedliche Verbindungen extrahiert werden können. Die einzelnen Komponenten der Vorrichtung können in Edelstahl, Borosilikatglas oder für die Rückstandsanalytik von polaren organischen Verbindungen geeignete Kunststoffe wie zum Beispiel PEEK, PTFE oder ähnlichen ausgeführt sein. Die redundante Ausführung der Filter und Sorbentien in der ersten Einrichtung zur Extraktion 100 und der zweiten Einrichtung zur Extraktion 300 mit der Möglichkeit der zeit- bzw. ereignisgesteuerten Umschaltung, zum Beispiel auf Grund einer Verstopfung, ermöglichen den kontinuierlichen Betrieb der Vorrichtung und gewährleisten somit eine zuverlässige Probenahme und Analyse. Die nicht dargestellte Steuerungseinrichtung kann zur Kontrolle aller Systemfunktionen, wie z. B. zur Druckkontrolle und/oder Durchflusskontrolle dienen sowie zu Übermittlung und gegebenenfalls Nachregelung von Druck- und Durchflussraten, sowie auch des pH-Wertes. Dabei kann eine Fernsteuer- bzw. Alarmfunktion über ein GSM-Modul oder eine Internetschnittstelle eingerichtet sein. Die erfindungsgemäße Vorrichtung lässt sich, insbesondere bei Realisierung in einen modularen Aufbau, in einfacher Weise skalieren und demzufolge einer gewünschten Probenahmekapazität bzw. Extraktionskapazität anpassen.

### Bezugszeichenliste

- Siebkorb: 1
- Zuleitung: 2
- Entspannungseinrichtung: 3
- Strömungsrichtung: 5
- Dosiergefäß: 10
- Abschaltelektrode: 11
- Füllrohr: 12
- Absperrhahn: 20
- Behälter: 40
- Leitfähigkeitselektrode: 41
- erste Einrichtung zur Extraktion: 100
- Kugelhahn: 120
- Filterkerze: 130
- Filterkartusche zur Feinfilterung: 140
- Sorbens: 150
- Zurückhalteeinrichtung: 160
- Siphonauslauf: 161
- Einrichtung zum Ansäuern/Alkalisieren: 200
- Mischeinrichtung: 210
- Säure-/Basebehälter: 220
- Dosiereinrichtung: 230
- Rührwerk: 240
- pH-Messeinrichtung: 250
- Füllstandsanzeige: 260
- Überlauf: 270
- Ablaufventil: 275
- Behälter: 280
- Elektrode: 281
- zweite Einrichtung zur Extraktion: 300
- Kugelhahn: 320
- Sorbens: 330
- Überlauf: 340
- Membranpumpe: 600
- Ventilsystem: 610
- Membranpumpe: 700
- Umschaltventil: 710
- Druckminderer: 720

## Patentansprüche

1. Verfahren zur Festphasenextraktion aus einer Flüssigkeit, bei dem die Flüssigkeit einer ersten Extraktion mittels eines Sorbens unterzogen wird, danach der pH-Wert der Flüssigkeit erniedrigt oder erhöht wird, und die Flüssigkeit einer zweiten Extraktion mittels eines Sorbens unterzogen wird.

2. Verfahren zur Festphasenextraktion aus einer Flüssigkeit nach Anspruch 1, wobei die Flüssigkeit einer ersten Extraktion mittels eines Sorbens unterzogen wird, danach die Flüssigkeit angesäuert oder alkalisiert wird, und die angesäuerte oder alkalisierte Flüssigkeit einer zweiten Extraktion mittels eines Sorbens unterzogen wird.

3. Verfahren zur Festphasenextraktion nach Anspruch 1 oder 2, **dadurch gekennzeichnet, dass** die Sorbentien mit den aus der Flüssigkeit extrahierten Substanzen einer Analyse zugeführt werden.

4. Verfahren zur Festphasenextraktion nach wenigstens einem der vorhergehenden Ansprüche, **dadurch gekennzeichnet, dass** innerhalb der ersten und/ oder zweiten Extraktion und vor der mittels eines Sorbens durchgeführten Extraktion eine Filterung, vorzugsweise mittels einer Filterkerze, durchgeführt wird.

5. Verfahren zur Festphasenextraktion nach wenigstens einem der vorhergehenden Ansprüche, **dadurch gekennzeichnet, dass** vor der ersten Extraktion eine Abscheidung von in der Flüssigkeit enthaltenen Schwebstoffen und/ oder absetzbaren Stoffen erfolgt.

6. Verfahren zur Festphasenextraktion nach wenigstens einem der vorhergehenden Ansprüche, **dadurch gekennzeichnet, dass** die Flüssigkeit Wasser ist und in der ersten Filterung einen pH-Wert zwischen 6 und 8 aufweist.

7. Verfahren zur Festphasenextraktion nach wenigstens einem der vorhergehenden Ansprüche, **dadurch gekennzeichnet, dass** nach der ersten Extraktion eine Bestimmung des pH-Wertes der Flüssigkeit durchgeführt wird, und durch Ansäuern oder Alkalisieren der Flüssigkeit ein bestimmter pH-Wert im sauren oder basischen Bereich eingestellt wird.

8. Vorrichtung zur Festphasenextraktion aus einer Flüssigkeit, umfassend eine erste Einrichtung zur Extraktion der Flüssigkeit mittels eines Sorbens, eine Einrichtung zur Änderung des pH-Wertes, sowie eine zweite Einrichtung zur Extraktion der Flüssigkeit mittels eines Sorbens, wobei die erste Einrichtung zur Extraktion der Flüssigkeit strömungstechnisch mit der Einrichtung zur Änderung des pH-Wertes und diese wiederum strömungstechnisch mit der zweiten Einrichtung zur Extraktion der Flüssigkeit verbunden ist.

9. Vorrichtung zur Festphasenextraktion nach Anspruch 8, **dadurch gekennzeichnet, dass** die erste Einrichtung zur Extraktion der Flüssigkeit in Strömungsrichtung vor dem Sorbens eine Filterkerze zur Entfernung von Schwebstoffen aus der Flüssigkeit aufweist.

10. Vorrichtung zur Festphasenextraktion nach wenigstens einem der Ansprüche 8 und 9, **dadurch gekennzeichnet, dass** in Strömungsrichtung vor wenigstens einer Extraktionseinrichtung eine Entspannungseinrichtung zur Abscheidung von absetzbaren Stoffen aus der Flüssigkeit angeordnet ist.

11. Vorrichtung zur Festphasenextraktion nach wenigstens einem der Ansprüche 8 bis 10, **dadurch gekennzeichnet, dass** die Vorrichtung in Strömungsrichtung nach der ersten Extraktionseinrichtung eine Mischeinrichtung umfasst und weiterhin eine Dosiereinrichtung umfasst, die zur Zugabe von Säure oder Base in die Mischeinrichtung eingerichtet ist.

12. Vorrichtung zur Festphasenextraktion nach wenigstens einem der Ansprüche 8 bis 11, **dadurch gekennzeichnet, dass** die erste Extraktionseinrichtung eine Filterkartusche zur Feinfilterung und eine mit dem Sorbens zumindest teilweise befüllte Kartusche zur Festphasenextraktion aufweist.

13. Vorrichtung zur Festphasenextraktion nach wenigstens einem der Ansprüche 8 bis 12, **dadurch gekennzeichnet, dass** die erste und/ oder zweite Extraktionseinrichtung eine Zurückhalteeinrichtung zur Zurückhaltung von Flüssigkeit in der Extraktionseinrichtung aufweist, die verhindert, dass unter Umgebungsdruck stehende Flüssigkeit in der Extraktionseinrichtung unter die geodätische Höhe des höchsten Punktes eines Filters oder Sorbens der Extraktionseinrichtung absinkt.

14. Vorrichtung zur Festphasenextraktion nach wenigstens einem der Ansprüche 8 bis 13, **dadurch gekennzeichnet, dass** die erste und/ oder zweite Extraktionseinrichtung eine Füllstandserfassungseinheit aufweist, mittels derer in der Extraktionseinrichtung ein bestimmter Füllstand der Flüssigkeit detektierbar ist.

15. Verwendung der Vorrichtung zur Festphasenextraktion gemäß einem der Ansprüche 8 bis 14 zur Gewinnung von Wasserproben aus Gewässern, Grundwässern, oder Abwässern sowie zur Extraktion von Substanzen aus den Proben zwecks Analyse.
